(19) Europäisches Patentamt
European Patent Office
Office européen des brevets

(11) EP 0 465 998 B1

(12) # EUROPÄISCHE PATENTSCHRIFT

(45) Veröffentlichungstag und Bekanntmachung des Hinweises auf die Patenterteilung:
**06.03.1996 Patentblatt 1996/10**

(51) Int Cl.$^6$: **C07H 15/203**, C12Q 1/34, G01N 33/58

(21) Anmeldenummer: **91110965.0**

(22) Anmeldetag: **02.07.1991**

(54) **Neue Beta-Galactosidase-Substrate für den CEDIA**

Beta-galactosidase substrate for CEDIA assays

Substrats de bêta-galactosidase pour analyse CEDIA

(84) Benannte Vertragsstaaten:
**AT BE CH DE DK ES FR GB GR IT LI LU NL SE**

(30) Priorität: **03.07.1990 DE 4021063**

(43) Veröffentlichungstag der Anmeldung:
**15.01.1992 Patentblatt 1992/03**

(73) Patentinhaber: **BOEHRINGER MANNHEIM GMBH**
**D-68298 Mannheim (DE)**

(72) Erfinder:
- **Guder, Hans-Joachim, Dr. rer.nat.**
  **D-67269 Grünstadt (DE)**
- **Herrmann, Rupert, Dr. rer.nat.**
  **D-82362 Weilheim (DE)**
- **Zdunek, Dietmar, Dr. rer.nat.**
  **D-82327 Tutzing (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

(56) Entgegenhaltungen:
**EP-A- 0 133 329**
**EP-A- 0 263 435**
**EP-A- 0 292 169**
**EP-A- 0 413 561**
**EP-A- 0 416 514**
**WO-A-86/02666**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 465 998 B1

## Beschreibung

Die Messung niedrigkonzentrierter Analyten in Körperflüssigkeiten wie z.B. Blut, Serum oder Urin erfordert hochempfindliche Testmethoden, wie sie z.B. in Immunoassays angeboten werden. Das Nachweissystem von bestimmten Immunotests beruht darauf, daß im Detektionsschritt die Reaktion des Markerenzyms β-Galactosidase mit einem chromogenen Enzymsubstrat erfolgt. Die Extinktion des freigesetzten Farbstoffs gibt dann einen direkten Hinweis auf die Menge des nachzuweisenden Analyten. Zur Erreichung hoher Sensitivität ist der Einsatz empfindlicher Enzymsubstrate erforderlich, die sowohl bezüglich ihrer enzymatischen Spaltung als auch in den spektralen Eigenschaften des freizusetzenden Chromophors den testspezifischen Anforderungen genügen.

Der CEDIA® (Cloned Enzyme-Donor Immunoassay) beruht auf der Assoziierung von zwei enzymatisch inaktiven β-Galactosidase-Fragmenten zu einem aktiven Gesamtenzym. Diese beiden Fragmente, ein sogenannter Enzymdonor und Enzymakzeptor, sind durch gentechnische Verfahren herstellbar. Für den CEDIA® geeignete Donoren und Akzeptoren sind im US-Patent 4,708,929 offenbart.

Der CEDIA® beruht darauf, daß ein Hapten kovalent an den Enzymdonor gebunden wird, und zwar auf eine Weise, daß die spontane Reassoziierung der Enzymfragmente zu einem aktiven Enzym nicht behindert wird. Bei Bindung eines haptenspezifischen Antikörpers an das Konjugat aus Hapten und Enzymdonor wird jedoch die Komplementation von Enzymdonor und,-akzeptor inhibiert. Der haptenspezifische Antikörper reguliert somit die Menge der sich bildenden aktiven β-Galactosidase. Diese wird photometrisch durch die Hydrolyse entsprechender chromogener Substrate quantitativ bestimmt. Eine ausführlichere Beschreibung des CEDIA®-Systems und seiner möglichen Anwendung ist einem Artikel von Khanna und Worthy (American Clinical Laboratory, October 1989) zu entnehmen.

Das Kriterium für die Qualität eines chromogenen Substrats für den CEDIA® wird anhand des dynamischen Meßbereiches ausgedrückt, der sich besonders durch die Steigung einer durch zwei Kalibratoren resultierenden Eichkurve beschreiben läßt. Dazu ist es wichtig, daß das Substrat (a) einen hohen Extinktionskoeffizienten und (b) eine hohe enzymatische Spaltrate durch die β-Galactosidase aufweist.

Das US-Patent 4,708,929 offenbart 2-Nitrophenyl-β-D-galactopyranosid als chromogenes Enzymsubstrat für den CEDIA®. Ein erheblicher Nachteil dieses Substrats ist jedoch seine geringe Sensitivität. Für die Bestimmung niedrigkonzentrierter Analyten in einer Lösung bestand daher ein Bedürfnis, verbesserte chromogene Substrate zu entwickeln.

Als verbesserte β-Galactosidase-Substrate werden in der EP-A 0 292 169 2-Halogen-4-nitrophenylgalactoside beschrieben. Für einen Einsatz im CEDIA® werden diese Substanzen allerdings nicht in Betracht gezogen.

EP-A-0 413 561 (veröffentlicht am 20.02.1991) beschreibt neue Substrate für β-Galactosidase. Diese Substrate besitzen die Formel:

(I) 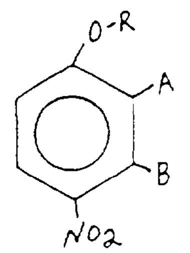

worin A Methoxyl oder Formyl und B Wasserstoff ist oder A Wasserstoff und B $NO_2$ oder CN ist und R eine β-D-Galactosidyl-Gruppe ist.

Aufgabe der vorliegenden Erfindung war es, verbesserte β-Galactosidasesubstrate, insbesondere zur Verwendung im CEDIA® zu entwickeln.

Erfindungsgemäß werden als verbesserte β-Galactosidase-Substrate Verbindungen der allgemeinen Formel I bereitgestellt,

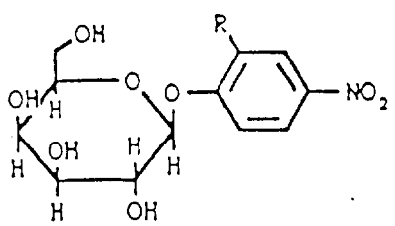 (I)

worin R $CF_3$ oder CN bedeutet. Die erfindungsgemäßen Verbindungen werden als 2-Trifluormethyl-4-nitrophenyl-β-D-galactopyranosid bzw. 2-Cyano-4-nitrophenyl-β-D-galactopyranosid bezeichnet.

Weiterhin ein Gegenstand der vorliegenden Erfindung ist ein Verfahren zur Herstellung einer erfindungsgemäßen Verbindung. Günstigerweise geht man von einer Ausgangsverbindung der allgemeinen Formel

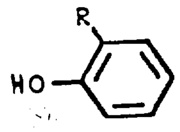

(II)

aus, worin R $CF_3$ oder CN bedeutet. In diese Verbindung II wird eine Nitrogruppe in p-Stellung zur Hydroxygruppe am aromatischen Ring eingeführt, so daß man eine Verbindung der allgemeinen Formel

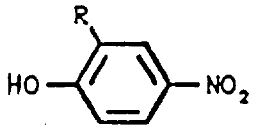

(III)

erhält. Die Verbindung III kann anschließend mit Silberoxid und Acetobromgalactose umgesetzt werden, wobei ein acetyliertes β-D-Galactopyranosid entsteht. Durch Abspaltung der Acetylgruppen erhält man das Endprodukt I.

Zur Herstellung von 2-Trifluormethyl-4-nitrophenyl-β-D-galactopyranosid nitriert man zunächst 2-Trifluormethylphenol mit konzentrierter Salpetersäure und gewinnt das entstehende 2-Trifluormethyl-4-nitrophenol. Dieses wird mit Silberoxid und Acetobromgalactose umgesetzt. Durch diese Reaktion entsteht ein acetyliertes Produkt, das durch Behandlung mit Alkali (z.B. Natriummethylat) unter Abspaltung der Acetylgruppen in das Endprodukt überführt werden kann.

2-Cyano-4-nitrophenyl-β-D-galactopyranosid ist durch ein ähnliches Verfahren erhältlich. Dazu geht man von 2-Hydroxybenzonitril aus, das zu 2-Hydroxy-5-nitro-benzonitril mit einem Gemisch aus konzentrierter Schwefel- und Salpetersäure umgesetzt wird. Das entstehende Produkt wird wiederum mit Silberoxid und Acetobromgalactose umgesetzt, wobei ein acetyliertes Zwischenprodukt entsteht. Daraus wiederum ist das Endprodukt durch Acetylgruppenabspaltung erhältlich.

Die erfindungsgemäßen Verbindungen sind insbesondere zur Verwendung als β-Galactosidase-Substrat geeignet. Dabei ist besonders bevorzugt, wenn die β-Galactosidase aus 2 Polypeptidfragmenten besteht, die nur bei Assoziation enzymatisch aktiv sind, während sie alleine im wesentlichen keine enzymatische Aktivität besitzen. Unter "im wesentlichen keine enzymatische Aktivität" ist zu verstehen, daß die enzymatische Restaktivität der einzelnen β-Galactosidase-Fragmente für eine Interferenz mit dem CEDIA® zu gering ist. Derartige β-Galactosidase-Donor- und Akzeptor-Fragmente sind im US-Patent 4,708,929 offenbart. Am meisten bevorzugt ist die Verwendung der erfindungsgemäßer Verbindungen als β-Galactosidase-Substrate in einem CEDIA®-system. Dabei ergeben sich die besten Ergebnisse in einem T4-CEDIA®-Test, wobei Thyroxin T4 als kovalent an den Enzymdonor gebundenes Hapten verwendet wird (siehe US-Patent 4,708,929).

Ein weiterer Gegenstand der Erfindung ist weiterhin ein Verfahren zum Nachweis eines Analyten in einer Probelösung mit einem CEDIA®-System, wobei man als Nachweisenzym β-Galactosidase und als β-Galactosidase-Substrat 2-Trifluormethyl-4-nitrophenyl-β-D-galactopyranosid oder/und 2-Cyano-4-nitrophenyl-β-D-galactopyranosid verwendet.

Ferner beinhaltet die Erfindung auch ein Reagenz zur Bestimmung eines Analyten mit einem CEDIA®-system, wobei man als β-Galactosidase-Substrat 2-Trifluormethyl-4-nitrophenyl-β-D-galactopyranosid oder/und 2-Cyano-4-nitrophenyl-β-D-galactopyranosid verwendet.

Vergleicht man die erfindungsgemäßen neuen Substrate mit dem im CEDIA® bislang eingesetzten o-Nitrophenyl-β-D-galactopyranosid, so findet man, daß die erfindungsgemäßen Substrate bezüglich ihrer enzymatischen Spaltrate und der Sensitivität gegenüber dem Substrat gemäß dem Stand der Technik deutlich überlegen sind.

Weiterhin wurde festgestellt, daß die erfindungsgemäßen Substrate auch dem 2-Chlor-4-nitrophenyl-β-D-galactopyranosid gemäß EP-A 0 292 169 überlegen sind.

Die Erfindung soll im weiteren durch die folgenden Beispiele erläutert werden.

Beispiel 1

Herstellung von 2-Cyano-4-nitrophenyl-β-D-galactopyranosid

1) **2-Bydroxy-5-nitro-benzonitril**

Zu 24 g (0.2 mol) 2-Hydroxy-benzonitril in 20 ml Wasser tropft man innerhalb von einer Stunde bei 10 °C eine Lösung aus 70 ml 40 %iger Salpetersäure und 10 ml konz. Schwefelsäure. Anschließend rührt man 24 Stunden bei 20 °C und schüttelt dann mit 100 ml Ethylacetat aus. Nach dem Eindampfen des organischen Lösungsmittels wird der feste Rückstand noch 2x mit je 500 ml kochendem n-Hexan extrahiert. Die Aufreinigung erfolgt durch Säulenchromatographie an Kieselgel (Kieselgel 60; Elutionsmittel Ethylacetat).

Nach Eindampfen des Lösungsmittels und Kristallisation aus Ethylacetat/Toluol erhält man 2 g des Produktes als

weiße Kristalle.

DC (Kieselgel 60; Ethylacetat): RF = 0.11
UV-Spektrum (in 0.1 M Kaliumphosphatpuffer pH 7.0): $\lambda_{max}$ = 376 nm ( $\varepsilon$ = 15700 l/molcm)

2) **2-Cyano-4-nitrophenyl 2,3,4,6-tetra-O-acetyl-β-D-galactopyranosid**

Eine Lösung aus 1 g (6.1 mmol) 2-Hydroxy-5-nitro-benzonitril in 200 ml trockenem Acetonitril wird mit 1.5 g (6.6 mmol) Silberoxid versetzt und 3 Stunden bei 20°C gerührt. Anschließend gibt man bei gleicher Temperatur eine Lösung von 2.7 g (6.6 mmol) Acetobromgalactose in 80 ml trockenem Acetonitril dazu und läßt 16 Stunden bei Raumtemperatur rühren. Die Silbersalze filtriert man über einen Seitzfilter ab und dampft das Filtrat zur Trockne ein. Das Rohprodukt wird aus Aceton/Wasser umkristallisiert.

Ausbeute : 2.9 g (96 %)
DC (Kieselgel 60; Toluol/Ethylacetat = 2/1) : RF = 0.27

3) **2-Cyano-4-nitrophenyl-β-D-galactopyranosid**

2.9 g (5.8 mmol) des acetylierten Produktes aus 2) wird in 10 ml absolutem Methanol gelöst und mit 4 ml einer gesättigten Natriummethylatlösung versetzt. Man läßt bis zur vollständigen Umsetzung (ca. 1 Stunde, DC-Kontrolle) bei 20°C rühren, filtriert den Niederschlag ab, wäscht mit wenig kaltem Methanol und trocknet bei 40°C im Vakuum.

Ausbeute : 1.0 g (53 %)
DC (Kieselgel 60; Chloroform/Methanol = 3/1) : RF = 0.35
1H-NMR (100 MHz, DMSO-d6) : $\delta$ (ppm) = 8.70 (d, J = 3 Hz; 1H), 8.49 (dd, J = 3 Hz, 9 Hz; 1H), 7.55 (d, J = 9 Hz; 1H), 5.36 - 5.26 (m; 2H), 4.94 (d, J = 7 Hz; 1H), 4.70 - 4.60 (m; 2H), 3.75 - 3.40 (m, 6H).
Massenspektrum: m/e = 326 (neg. FAB)

Beispiel 2

Herstellung von 2-Trifluormethyl-4-nitrophenyl-β-D-galactopyranosid

1) **2-Trifluormethyl-4-nitrophenol**

Zu einer Suspension aus 10 g (0.06 mol) 2-Trifluormethylphenol in 30 ml Wasser gibt man unter Eiskühlung innerhalb einer Stunde 20 ml (0.18 mol) 40 %ige wässrige Salpetersäure. Man läßt noch 2 Stunden bei 20 ° C rühren und schüttelt anschließend mit 100 ml Ethylacetat aus. Nach Trocknen der organischen Phase über Natriumsulfat wird das Lösungsmittel am Rotationsverdampfer abgezogen und das Produkt mittels Säulenchromatographie über Kieselgel aufgereinigt (Kieselgel 60; Elutionsmittel Ethylacetat/Petrolether = 1/3). DC (Kieselgel 60; Ethylacetat/Toluol = 1/2) : RF = 0.44 Die Fraktionen werden gesammelt und am Rotationsverdampfer unter Vakuum eingedampft. Zur weiteren Aufreinigung und Kristallisation wird das Produkt in 100 ml Wasser gelöst und mit 1 N HCl auf pH 2 gestellt. Nach 24 Stunden bei 4°C werden die ausgefallenen Kristalle abfiltriert, mit wenig kaltem Wasser gewaschen und bei 40°C unter Vakuum getrocknet. Ausbeute : 2.3 g (19 %).

DC : (Kieselgel 60; Ethylacetat): RF = 0.11
UV-Spektrum (in 0.1 M Kaliumphosphatpuffer pH 7.0): $\lambda_{max}$ = 384 nm ($\varepsilon$= 18800 l/molcm).

2) **2-Trifluormethyl-4-nitrophenyl 2,3,4,6-tetra-o-acetyl-β-D-galactopyranosid**

Die Herstellung erfolgt aus 2 g (9.7 mmol) 2-Trifluormethyl-4-nitrophenol, 4.11 g (10 mmol) Acetobromgalactose und 2.32 g (10 mmol) Silberoxid analog Beispiel 1.2.

Ausbeute : 2 g (38%).
DC (Kieselgel 60, Toluol/Ethylacetat = 2/1) : RF = 0.35

3) **2-Trifluormethyl-4-nitrophenyl-β-D-galactopyranosid**

2.0 g (3.7 mmol) der acetylierten Verbindung aus 2) wird in 10 ml absolutem Methanol gelöst und mit 4 ml einer

gesättigten Natriummethylatlösung versetzt. Man läßt 1 Stunde bei 20°C rühren, neutralisiert mit saurem Ionenaustauscher (Dowex® 50 WX 8, H+) und dampft im Vakuum bis zur Trockne ein. Die Aufreinigung erfolgt durch Säulenchromatographie an Kieselgel (Elutionsmittel Chloroform/Methanol = 3/1).

Ausbeute : 0.25 g (18 %).
DC (Kieselgel 60; Chloroform/Methanol = 3/1) : RF = 0.5
1H - NMR (100 MHz, DMSO - d6) : δ (ppm) = 8.50 (dd, J = 3 Hz, 9 Hz; 1H), 8.39 (d, J = 3 Hz; 1H), 7.58 (d, J = 9 Hz; 1H), 5.30 - 5.05 (m; 2H), 5.03 - 4.83 (m; 1H), 4.80 - 4.55 (m; 2H), 3.82 - 3.40 (m; 6H)

Beispiel 3

Vergleich der erfindungsgemäßen Substrate 2-Cyano-4-nitrophenyl-β-D-galactopyranosid (2-CN-4-NPG), 2-Trifluormethyl-4-nitrophenyl-β-D-galactopyranosid (2-CF3-4-NPG) mit den bereits bekannten Substraten 2-Chlor-4-nitrophenyl-β-D-galactopyranosid (2-Cl-4-NPG) und 2-Nitrophenyl-β-D-galactopyranosid (oNPG)

1. Wellenlängen der Absorptionsmaxima der Farbstoffe und die zugehörigen Extinktionskoeffizienten (ε)

| Farbstoff | lambda max (nm) | ε(l/mol cm) |
|---|---|---|
| 2-Cl-4-Nitrophenol | 405 | 16600 |
| 2-CN-4-Nitrophenol | 376 | 15700 |
| 2-CF3-4-Nitrophenol | 384 | 18800 |
| 2-Nitrophenol | 408 | 2040 |

gemessen in 0.1 mol/l Kaliumphosphatpuffer, pH 7.0 am Absorptionsmaximum des Farbstoffs

2. Sensitivitätsvergleich der Substrate bzw. ihrer enzymatischen Spaltrate

Ermittelt wurde die Extinktionsänderung pro Minute (mE / min) im Bereich des Absorptionsmaximums (s.Beispiel 3.1) des jeweiligen Farbstoffes unter Verwendung identischer Mengen des Enzyms β-Galactosidase, genaue Bedingungen siehe Beispiel 4 Punkt II) a) und b).

| Substrat | mE / min | Messwellenlänge (nm) |
|---|---|---|
| 2-Cl-4-NPG | 0.085 | 405 |
| 2-CN-4-NPG | 0.150 | 376 |
| 2-CF3-4-NPG | 0.109 | 384 |
| oNPG | 0.024 | 408 |

3. Bewertung im T4/CEDIA® (CEDIA® mit Thyroxin T4 als Hapten und monoklonalen Antikörpern gegen Thyroxin T4)

Die Testbedingungen waren wie in Beispiel 4, Punkt I) a) und b). Kriterium für die Bewertung der einzelnen Substrate war die Steigung der Eichkurve. Diese wurde wie in Beispiel 4, Punkt III) a) beschrieben, ermittelt.

| Substrat | rel. Steigung | Messwellenlänge (nm) |
|---|---|---|
| 2-Cl-4-NPG | 494 | 415 |
| 2-CN-4-NPG | 561 | 376 |
| 2-CF3-4-NPG | 528 | 376 |
| oNPG | 100 | 415 |

Beispiel 4

UNTERSUCHUNG VON SUBSTRAT-ANALOGEN FÜR CEDIA® TESTS

Methoden

Zwei Testmethoden wurden verwendet.

1) Aktivität im T4/CEDIA®-Test,
2) Wirksamkeit als Substrate für β-Galactosidase.

I) Durchführung des T4/CEDIA®-Tests

(Definitionen: EA = Enzym Acceptor: ED = Enzym Donor; $EA_{22}$ = klonierter Enzym-Akzeptor für CEDIA®; $ED_4$ = klonierter Enzym Donor für CEDIA®)

Die Begriffe $EA_{22}$ und $ED_4$ werden gemäß US-Patent 4,708,929 verwendet. Dort sind auch die Aminosäure-Sequenzen dieser Polypeptide offenbart.

a) Reagenzien

1) EA Puffer:- $NaH_2PO_4$ (20 mmol/l), $Na_2HPO_4$ (30 mmol/l), [Ethylenbis(oxyethylennitrilo)]tetraessigsäure (EGTA) (18 mmol/l), Sucrose (25 mmol/l), $Mg(OAc)_2$ (9.67 mmol/l), 4-Morpholinpropansulfonsäure (MOPS) (150 mmol/l), NaCl (400 mmol/l), L-Methionin (10 mmol/l), Pluronic L-101 (0.05%), Tween® 20 (0.05%)m, Hydroxypropyl-β-Cyclodextrin (0.2%), Dithiothreitol (DTT) (0.05 mmol/l), $NaN_3$ (23 mmol/l). pH 7.4.

2) ED Puffer:- $NaH_2PO_4$ (42 mmol/l), $Na_2HPO_4$ (8 mmol/l), NaCl (400 mmol/l), MOPS (0.02 mmol/l), EGTA (10 mmol/l), Tween 20 (0.05%), Pluronic® L-101 (0.05%), N-Lauroylsarcosin (0.03%), DTT (0.05 mmol/l), $NaN_3$ (23 mmol/l). pH 6.5.

3) EA Reagenz:- Im EA Puffer wurden 546 U/ml $EA_{22}$, 63 nmol/l monoklonaler Antikörper <T4> (kommerziell erhältlich z.B. von Western Chemical Research Corp., Denver, Colorado, USA, siehe US 4,708,929) und 800 µmol/l ANS (8-Anilinonaphthalin-1-sulfonsäure, Ammoniumsalz) eingesetzt.

4) ED Reagenz:- Im ED Puffer wurden als Analyt 115 nmol/l $ED_4$-Thyroxin T4 Konjugat (Herstellung entsprechend ED-Thyroxin-Konjugat nach US-Patent 4,708,929), und das jeweils getestete β-Galactosidase-Substrat (1 mg/ml) eingesetzt.

b) Methode

Der T4/CEDIA® wurde auf einem Hitachi 704 Automatic Analyzer mit den folgenden Reagenzmengen und unter den angegebenen Bedingungen durchgeführt:

| | |
|---|---|
| Probenvolumen: | 12 µl |
| Reagenz 1 ($ED_4$-Konjugat + Substrat): | 235 µl |
| Reagenz 2 ($EA_{22}$ + MAK): | 135 µl |
| Gesamtvolumen: | 382 µl |
| Meßwellenlänge: | 376/415nm |
| Temperatur: | 37°C |

ΔE/min wurde im Intervall 9-10 Min. gemessen.

II) Enzymaktivität

a) Reagenzien

1) 0.05M Kaliumphosphat-Puffer, pH 7.0.

2) 10mmol/l Magnesiumchlorid-Lösung

3) 69.8% (v/v) Mercaptoethanol-Lösung

4) β-Galactosidase (Boehringer Mannheim GmbH, Bestell. Nr. 105 031), 0.3U/ml in Lösung a)

5) Substrat, 5.9 mg/ml in Lösung a)

b) Methode

Vorinkubation: folgende Substanzen wurden in Plastik-Küvetten pipettiert:

| | | Leerwert | Probe |
|---|---|---|---|
| Puffer | (1) | 1100 µl | 1100 µl |
| $MgCl_2$ | (2) | 150 µl | 150 µl |
| Substrat | (5) | 200 µl | 200 µl |
| Mercaptoethanol | (3) | 15 µl | 15 µl |

Nach dem Mischen wurde die Reaktion gestartet:

| | | | |
|---|---|---|---|
| Puffer | (1) | 25 µl | -- |
| β-Galactosidase | (4) | -- | 25 µl |

ΔE/min wurde aus dem linearen Bereich berechnet

III) Auswertung der Test-Ergebnisse

a) CEDIA®-Test

Für alle Substrate wurden Eichkurven produziert. Von diesen Kurven wurden die Steigungen verglichen.

$$\text{Steigung} = \frac{\text{High Kalibrator Rate-Low Kalibrator Rate}}{\text{Konz. High Kalibrator-konz. Low Kalibrator}}$$

Steigung ausgedrückt als prozentualer Wert des 2-Nitrophenyl-β-galactosids (=100%) für T4/CEDIA®

**Patentansprüche**

**Patentansprüche für folgende Vertragsstaaten: AT, BE, CH, LI, DE, DK, FR, GB, GR, IT, LU, NL, SE**

1. Verbindung der allgemeinen Formel

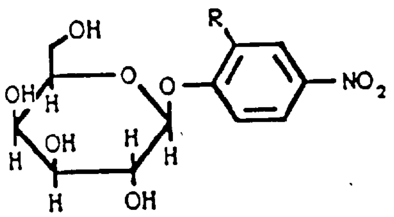

(I)

worin R $CF_3$ oder CN bedeutet.

**2.** Verfahren zur Herstellung einer Verbindung nach Anspruch 1,
**dadurch gekennzeichnet,** daß man eine Ausgangsverbindung der allgemeinen Formel

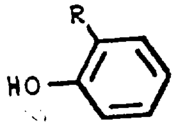

(II)

worin R $CF_3$ oder CN bedeutet, durch Nitrierung am aromatischen Ring in eine Verbindung der Formel

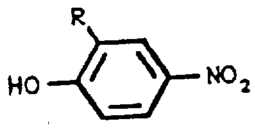

(III)

überführt, die entstehende Verbindung III mit Silberoxid und Acetobromgalactose umsetzt und das Endprodukt

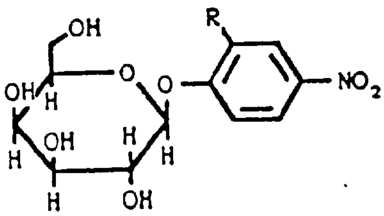

durch Acetylgruppen-Abspaltung gewinnt.

**3.** Verfahren nach Anspruch 2 zur Herstellung von 2-Trifluormethyl-4-nitrophenyl-β-D-galactopyranosid,
**dadurch gekennzeichnet,** daß man als Ausgangsverbindung 2-Trifluormethylphenol einsetzt.

**4.** Verfahren nach Anspruch 2 zur Herstellung von 2-Cyano-4-nitro-phenyl-β-D-galactopyranosid,
**dadurch gekennzeichnet,** daß man als Ausgangsverbindung 2-Hydroxybenzonitril einsetzt.

**5.** Verwendung einer Verbindung nach Anspruch 1 als β-Galactosidase-Substrat.

**6.** Verwendung nach Anspruch 5,
**dadurch gekennzeichnet,** daß die β-Galactosidase aus zwei Polypeptid-Fragmenten besteht, die bei Assoziation enzymatisch aktiv sind, die aber alleine im wesentlichen keine enzymatische Aktivität besitzen.

**7.** Verwendung nach Anspruch 6 in einem CEDIA®-System.

**8.** Verfahren zum Nachweis eines Analyten in einer Probelösung mit einem CEDIA®-System, wobei man als Nachweisenzym β-Galactosidase verwendet,
**dadurch gekennzeichnet,** daß man als β-Galactosidase-Substrat 2-Trifluormethyl-4-nitrophenyl-β-D-galactopyranosid oder/und 2-Cyano-4-nitrophenyl-β-D-galactopyranosid verwendet.

**9.** Reagenz zum Nachweis eines Analyten in einer Probelösung mit einem CEDIA®-System, wobei das Nachweisenzym β-Galactosidase ist,
**dadurch gekennzeichnet**, daß es als β-Galactosidase-Substrat 2-Trifluormethyl-4-nitrophenyl-β-D-galactopyranosid oder/und 2-cyano-4-nitrophenyl-β-D-galactopyranosid enthält.

**Patentansprüche für folgenden Vertragsstaat: ES**

1. Verfahren zur Herstellung einer Verbindung der allgemeinen Formel

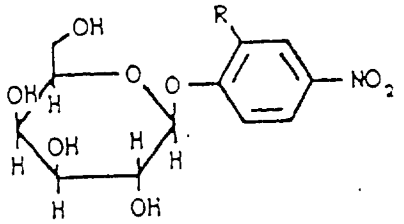

(I)

worin R $CF_3$ oder CN bedeutet,
**dadurch gekennzeichnet,**
daß man eine Ausgangsverbindung der allgemeinen Formel

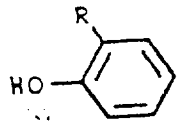

(II)

worin R $CF_3$ oder CN bedeutet, durch Nitrierung am aromatischen Ring in eine Verbindung der Formel

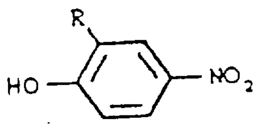

(III)

überführt, die entstehende Verbindung III mit Silberoxid und Acetobromgalactose umsetzt und das Endprodukt

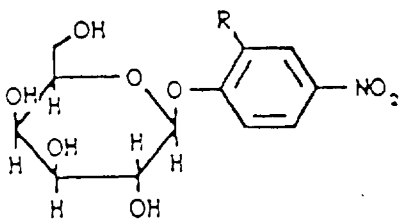

durch Acetylgruppen-Abspaltung gewinnt.

2. Verfahren nach Anspruch 1 zur Herstellung von 2-Trifluormethyl-4-nitrophenyl-β-D-galactopyranosid,
**dadurch gekennzeichnet,**
daß man als Ausgangsverbindung 2-Trifluormethylphenol einsetzt.

3. Verfahren nach Anspruch 1 zur Herstellung von 2-Cyano-4-nitro-phenyl-β-D-galactopyranosid,
**dadurch gekennzeichnet,**
daß man als Ausgangsverbindung 2-Hydroxybenzonitril einsetzt.

4. Verwendung einer nach Anspruch 1 hergestellten Verbindung als β-Galactosidase-Substrat.

5. Verwendung nach Anspruch 4,
**dadurch gekennzeichnet,**
daß die β-Galactosidase aus zwei Polypeptid-Fragmenten besteht, die bei Assoziation enzymatisch aktiv sind, die aber alleine im wesentlichen keine enzymatische Aktivität besitzen.

6. Verwendung nach Anspruch 5 in einem CEDIA®-System.

7. Verfahren zum Nachweis eines Analyten in einer Probelösung mit einem CEDIA®-System, wobei man als Nachweisenzym β-Galactosidase verwendet,
**dadurch gekennzeichnet,**
daß man als β-Galactosidase-Substrat 2-Trifluormethyl-4-nitrophenyl-β-D-galactopyranosid oder/und 2-Cyano-4-nitrophenyl-β-D-galactopyranosid verwendet.

**8.** Reagenz zum Nachweis eines Analyten in einer Probelösung mit einem CEDIA®-System, wobei das Nachweisenzym β-Galactosidase ist,
**dadurch gekennzeichnet,**
daß es als β-Galactosidase-Substrat 2-Trifluormethyl-4-nitrophenyl-β-D-galactopyranosid oder/und 2-Cyano-4-nitrophenyl-β-D-galactopyranosid enthält.

## Claims

### Claims for the following Contracting States : AT, BE, CH,LI, DE, DK, FR, GB, GR, IT, LU NL, SE

**1.** Compound having the general formula

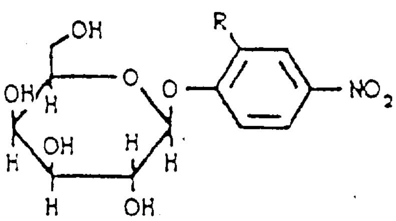

(I)

wherein R denotes $CF_3$ or CN.

**2.** Process for the preparation of a compound according to claim 1, **characterized in that** a starting compound of the general formula

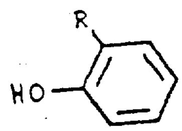

(II)

wherein R denotes $CF_3$ or CN is converted by nittration on the aromatic ring into a compound of the formula

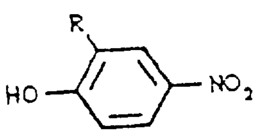

(III)

in that the resultant compound III is reacted with silver oxide and acetobromogalactose and in that the end product

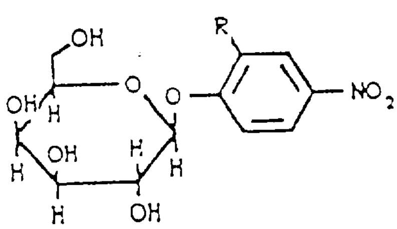

is recovered by splitting off acetyl groups.

**3.** Process according to claim 2 for the preparation of 2-trifluoromethyl-4-nitrophenyl-β-D-galactopyranoside, **characterized in that** 2-trifluoromethylphenol is used as a starting material.

**4.** Process according to claim 2 for the preparation of 2-cyano-4-nitrophenyl-β-D-galactopyranoside, **characterized in that** 2-hydroxybenzonitrile is used as a starting material.

**5.** Use of a compound according to claim 1 as a β-galactosidase substrate.

**6.** Use according to claim 5, **characterized in that** the β-galactosidase consists of two polypeptide fragments which, in the case of association, are enzymatically active, but which alone possess substantially no enzymatic activity.

7. Use according to claim 6 in a CEDIA® system.

8. Process for the detection of an analyte in a sample solution with a CEDIA® system using β-galactosidase as a detection enzyme **characterized in that** as β-galactosidase substrate there is used 2-trifluoromethyl-4-nitrophenyl-β-D-galactopyranoside and/or 2-cyano-4-nitrophenyl-β-D-galactopyranoside.

9. Reagent for the detection of an analyte in a sample solution with a CEDIA® system using β-galactosidase as a detection enzyme **characterized in that** as β-galactosidase substrate it contains 2-trifluoromethyl-4-nitrophenyl-β-D-galactopyranoside and/or 2-cyano-4-nitrophenyl-β-D-galactopyranoside.

**Claims for the following Contracting State: ES**

1. Process for the preparation of a compound having the general formula

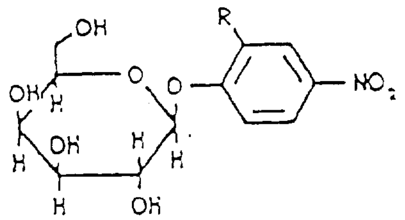

(I)

wherein R denotes $CF_3$ or CN, **characterized in that** a starting compound of the general formula

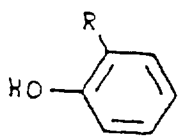

(II)

wherein R denotes $CF_3$ or CN is converted by nitrtation on the aromatic ring into a compound of the formula

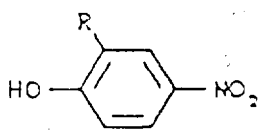

(III)

in that the resultant compound III is reacted with silver oxide and acetobromogalactose and in that the end product

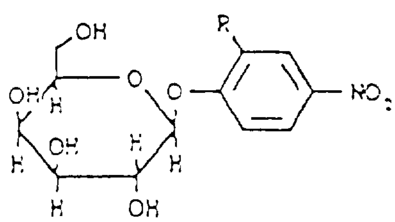

is recovered by splitting off acetyl groups.

2. Process according to claim 1 for the preparation of 2-trifluoromethyl-4-nitrophenyl-β-D-galactopyranoside, **characterized in that** 2-trifluoromethylphenol is used as a starting material.

3. Process according to claim 1 for the preparation of 2-cyano-4-nitrophenyl-β-D-galactopyranoside, **characterized in that** 2-hydroxybenzonitrile is used as a starting material.

4. Use of a compound prepared according to claim 1 as a β-galactosidase substrate.

5. Use according to claim 4, **characterized in that** the β-galactosidase consists of two polypeptide fragments which, in the case of association, are enzymatically active, but which alone possess substantially no enzymatic activity.

6. Use according to claim 5 in a CEDIA® system.

7. Process for the detection of an analyte in a sample solution with a CEDIA® system using β-galactosidase as a detection enzyme **characterized in that** as β-galactosidase substrate there is used 2-trifluoromethyl-4-nitrophenyl-β-D-galactopyranoside and/or 2-cyano-4-nitrophenyl-β-D-galactopyranoside.

8. Reagent for the detection of an analyte in a sample solution with a CEDIA® system using β-galactosidase as a detection enzyme **characterized in that** as β-galactosidase substrate it contains 2-trifluoromethyl-4-nitrophenyl-β-D-galactopyranoside and/or 2-cyano-4-nitrophenyl-β-D-galactopyranoside.

**Revendications**

**Revendications pour les Etats contractants suivants : AT, BE, CH, LI, DE, DK, FR, GB, GR, IT, LU, NL, SE**

1. Composé de formule générale

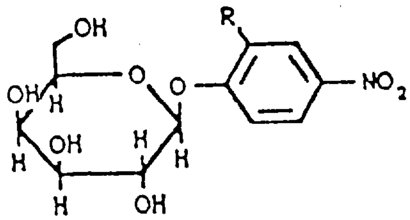

(I)

dans laquelle R représente $CF_3$ ou CN.

2. Procédé de préparation d'un composé selon la revendication 1, caractérisé en ce que l'on convertit un composé de départ de formule générale

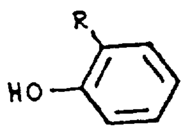

(II)

dans laquelle R représente CF3 ou CN par nitration sur le cycle aromatique en un composé de formule

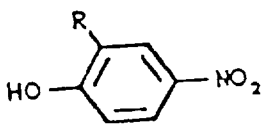

(III)

on fait réagir le composé III formé avec l'oxyde d'argent et l'acétobromogalactose et on obtient le produit final

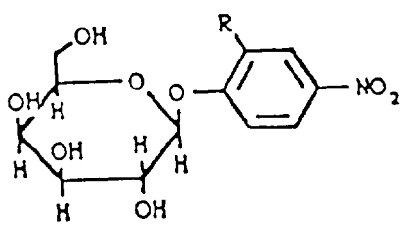

par clivage des groupes acétyle.

3. Procédé selon la revendication 2 pour la préparation du 2-trifluorométhyl-4-nitrophényl-β-D-galactopyranoside, caractérisé en ce que l'on utilise le 2-trifluorométhylphénol comme composé de départ.

4. Procédé selon la revendication 2 pour la préparation du 2-cyano-4-nitrophényl-β-D-galactopyranoside, caractérisé en ce que l'on utilise le 2-hydroxybenzonitrile comme composé de départ.

5. Utilisation d'un composé selon la revendication 1 comme substrat de la β-galactosidase.

**6.** Utilisation selon la revendication 5, caractérisée en ce que la β-galactosidase consiste en deux fragments polypeptidiques qui sont enzymatiquement actifs par association mais qui, seuls, ne possèdent sensiblement aucune activité enzymatique.

**7.** Utilisation selon la revendication 6 dans un système CEDIA®.

**8.** Procédé pour la mise en évidence d'un analyte dans une solution échantillon avec un système CEDIA®, dans lequel on utilise la β-galactosidase comme enzyme de mise en évidence, caractérisé en ce que l'on utilise le 2-trifluorométhyl-4-nitrophényl-β-D-galactopyranoside et/ou le 2-cyano-4-nitrophényl-β-D-galactopyranoside comme substrat de la β-galactosidase.

**9.** Réactif pour la mise en évidence d'un analyte dans une solution échantillon avec un système CEDIA®, dans lequel l'enzyme de mise en évidence est la β-galactosidase, caractérisé en ce qu'il contient du 2-trifluorométhyl-4-nitrophényl-β-D-galactopyranoside et/ou du 2-cyano-4-nitrophényl-β-D-galactopyranoside comme substrat de la β-galactosidase.

**Revendications pour l'Etat contractant suivant : ES**

**1.** Procédé de préparation d'un composé de formule générale

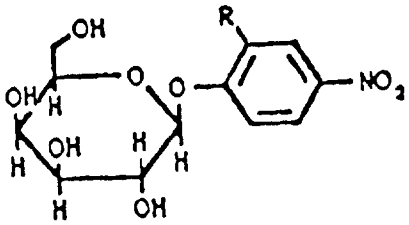

(I)

dans laquelle R représente $CF_3$ ou CN, caractérisé en ce que l'on convertit un composé de départ de formule générale

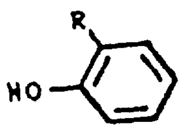

(II)

dans laquelle R représente $CF_3$ ou CN par nitration sur le cycle aromatique en un composé de formule

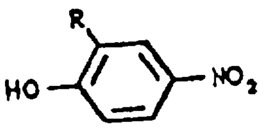

(III)

on fait réagir le composé III formé avec l'oxyde d'argent et l'acétobromogalactose et on obtient le produit final

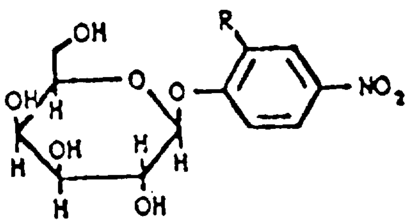

par clivage des groupes acétyle.

**2.** Procédé selon la revendication 1 pour la préparation du 2-trifluorométhyl-4-nitrophényl-β-D-galactopyranoside, caractérisé en ce que l'on utilise le 2-trifluorométhylphénol comme composé de départ.

**3.** Procédé selon la revendication 1 pour la préparation du 2-cyano-4-nitrophényl-β-D-galactopyranoside, caractérisé en ce que l'on utilise le 2-hydroxybenzonitrile comme composé de départ.

4. Utilisation d'un composé préparé selon la revendication 1 comme substrat de la β-galactosidase.

5. Utilisation selon la revendication 4, caractérisée en ce que la β-galactosidase consiste en deux fragments polypeptidiques qui sont enzymatiquement actifs par association mais qui, seuls, ne possèdent sensiblement aucune activité enzymatique.

6. Utilisation selon la revendication 5 dans un système CEDIA®.

7. Procédé pour la mise en évidence d'un analyte dans une solution échantillon avec un système CEDIA®, dans lequel on utilise la β-galactosidase comme enzyme de mise en évidence, caractérisé en ce que l'on utilise le 2-trifluorométhyl-4-nitrophényl-β-D-galactopyranoside et/ou le 2-cyano-4-nitrophényl-β-D-galactopyranoside comme substrat de la β-galactosidase.

8. Réactif pour la mise en évidence d'un analyte dans une solution échantillon avec un système CEDIA®, dans lequel l'enzyme de mise en évidence est la β-galactosidase, caractérisé en ce qu'il contient du 2-trifluorométhyl-4-nitrophényl-β-D-galactopyranoside et/ou du 2-cyano-4-nitrophényl-β-D-galactopyranoside comme substrat de la β-galactosidase.